Europäisches Patentamt

European Patent Office

Office européen des brevets

(19)

(11) Numéro de publication: **0 241 384 B1**

(12) **FASCICULE DE BREVET EUROPEEN**

(45) Date de publication de fascicule du brevet: **14.06.95**

(51) Int. Cl.6: **A61C 5/10**, A61C 13/083, A61C 13/00, A61C 5/08, A61K 6/06

(21) Numéro de dépôt: **87400822.0**

(22) Date de dépôt: **10.04.87**

(54) **Procédé de fabrication de prothèses dentaires et prothèses obtenues par ce procédé.**

(30) Priorité: **11.04.86 FR 8640078**

(43) Date de publication de la demande:
**14.10.87 Bulletin 87/42**

(45) Mention de la délivrance du brevet:
**14.06.95 Bulletin 95/24**

(84) Etats contractants désignés:
**AT BE CH DE ES FR GB GR IT LI LU NL SE**

(56) Documents cités:
CH-A- 428 088        DE-B- 1 491 042
DE-C- 216 213        FR-A- 2 129 058
FR-A- 2 291 736      US-A- 4 040 844
US-A- 4 562 882

CERAMIC ENGINEERING AND SCIENCE PRO-
CEEDINGS vol. 6 janvier/février 1985, pages 1
- 9, Columbus, Ohio; US; J.W. MACLEAN:
"Current Status and Future of Ceramics in
Dentistry"

(73) Titulaire: **Tyszblat, Michèle**
**3 Avenue Séverine**
**F-92400 Courbevoie (FR)**

(72) Inventeur: **Tyszblat, Michèle**
**3 Avenue Séverine**
**F-92400 Courbevoie (FR)**

(74) Mandataire: **Nony, Michel et al**
**NONY & ASSOCIES**
**29, rue Cambacérès**
**F-75008 Paris (FR)**

EP 0 241 384 B1

**EP 0 241 384 B1**

**Description**

La présente invention est relative à un nouveau procédé de fabrication de prothèses dentaires ainsi qu'aux prothèses obtenues par ce procédé.

Par prothèses dentaires, on entend de manière générale toutes pièces destinées à être placées sur la denture d'un patient dans le but de la restaurer en totalité ou en partie dans sa forme naturelle.

C'est ainsi que les prothèses fabriquées selon l'invention peuvent être par exemple des chapes périphériques ou couronnes qui viennent se placer sur le moignon d'une dent naturelle, ou bien des prothèses généralement désignées sous les termes de "inlay" et "onlay" qui sont destinées à reconstituer une altération partielle d'une dent en remplissant la cavité résultant de la perte de substance de la dent par une pièce de même forme réalisée par le prothésiste, ou bien encore des bridges qui sont des prothèses qui prennent appui simultanément sur les parties subsistantes d'au moins deux dents en compensant éventuellement une ou plusieurs dents manquantes.

On connaît déjà des procédés pour fabriquer de telles prothèses qui sont le plus souvent des pièces métalliques moulées par fusion, sur lesquelles on peut fixer à haute température un émail qui donne à la prothèse l'aspect d'une dent naturelle.

Ces prothèses de type connu présentent l'inconvénient d'être d'une fabrication délicate et coûteuse. De plus, du fait de leur composante métallique elles ne sont pas complètement biocompatibles et l'on observe à la longue des phénomènes de corrosion des prothèses qui conduisent au remplacement de ces dernières.

Le document CH-A-42 8088 décrit un procédé de fabrication de prothèses dentaires céramiques dans lesquelles l'oxyde métallique présente après cuisson une surface imperméable aux fluides de la bouche. Ces prothèses ont l'inconvénient de ne pouvoir être obtenues avec une précision de forme suffisante pour s'adapter correctement sur une dent préalablement préparée.

Le document DE-B-14 91 042 décrit un procédé de fabrication de dents artificielles pour lesquelles la précision dimensionnelle ne se pose pas non plus. Ces dents artificielles comportent des particules discontinues d'oxydes métalliques dispersées dans une masse continue de verre.

La présente invention vise à réaliser des prothèses dentaires qui sont de nature totalement céramique et qui possèdent de très bonnes caractéristiques mécaniques en ayant un aspect comparable à celui des dents naturelles.

Les prothèses fabriquées conformément à l'invention possèdent la compatibilité biologique des céramiques. Elles sont d'un coût de fabrication inférieur aux prothèses que l'on sait actuellement fabriquer et elles s'adaptent avec une très grande précision aux parties des dents naturelles sur lesquelles elles doivent être fixées.

On sait que par "céramiques" on entend dans l'art dentaire non seulement les produits réalisés en terre cuite mais aussi ceux qui comportent des émaux et des oxydes métalliques tels que par exemple l'oxyde d'aluminium ou l'oxyde de zirconium. On désigne par contre sous le terme de "prothèses céramo-métalliques" celles qui sont réalisées en partie à l'aide de métaux qui se trouvent généralement sous forme d'alliages.

La présente invention a pour objet un procédé pour fabriquer des prothèses dentaires céramiques, caractérisé par le fait que l'on réalise un modèle de la dent qui doit recevoir la prothèse dans une masse de moulage telle qu'on plâtre qui présente lors de sa solidification une légère expansion linéaire ; que l'on prépare une barbotine constituée par une suspension dans l'eau de particules d'oxydes métalliques tels que par exemple de l'oxyde d'aluminium et/ou de l'oxyde de zirconium, additionnée d'un agent de stabilisation de la suspension et éventuellement d'un agent de contrôle du pH ; que l'on met en contact le modèle de la dent précédemment réalisé, avec la barbotine, de telle sorte que les particules d'oxydes métalliques s'agglomèrent sur la surface du modèle de la dent, jusqu'à ce que l'on obtienne une épaisseur suffisante ; que l'on donne à la couche d'oxydes métalliques la forme extérieure que l'on désire conférer à l'infrastructure de la prothèse ; que l'on procède à la cuisson du modèle de la dent ainsi revêtu de l'infrastructure à base d'oxydes métalliques pour réaliser dans un premier temps une déshydratation du modèle de la dent qui provoque son retrait, puis un léger frittage en phase solide des particules d'oxydes métalliques ; et que l'on imprègne d'un verre l'infrastructure ainsi frittée à une température suffisante pour occuper la totalité des pores ouverts existant dans l'infrastructure.

Selon une variante préférée de l'invention, on procède ensuite au revêtement de la surface externe de la prothèse avec un émail compatible avec la nature du verre ayant servi à l'imprégnation de l'infrastructure et possédant un coefficient de dilatation voisin de celui de cette dernière.

On voit que le procédé conforme à l'invention consiste dans un premier temps à réaliser une infrastructure ou squelette qui est constitué par une agglomération de particules d'oxydes métalliques

2

réunies par un léger frittage en phase solide puis dans un second temps à garnir la totalité des interstices existants entre les particules d'oxydes métalliques à l'aide d'un verre fondu qui remplit complètement le réseau continu de cavités laissé par les particules d'oxydes métalliques frittées.

En d'autres termes, les prothèses selon l'invention sont constituées par imbrication complète de deux réseaux continus dont l'un est constitué par de fines particules d'oxydes métalliques frittées en phase solide et dont l'autre est constitué par du verre.

Les prothèses dentaires obtenues selon l'invention se sont révélées présenter d'excellentes caractéristiques mécaniques. D'une manière surprenante, leurs caractéristiques mécaniques sont en effet supérieures à celles que l'on peut obtenir par le frittage complet des particules d'oxydes métalliques sans les imprégner avec du verre.

La masse de moulage destinée, conformément à l'invention, à réaliser le modèle de la dent, peut être un plâtre de type alpha constitué par un hémi-hydrate de sulfate de calcium Ca SO$_4$, 1/2 H$_2$O sans aucune charge de nature minérale. Ce plâtre doit présenter une expansion linéaire de solidification (ou de prise) comprise de préférence entre 0,1 % et 0,4 %. Cette expansion croît avec la quantité de plâtre mélangée à une quantité d'eau donnée pour réaliser le moulage. Elle augmente également par adjonction de chlorure de sodium (ClNa) ou d'éther cellulosique.

Cette masse de moulage peut être également constituée par des charges réfractaires telles que de l'oxyde de silicium ou de l'oxyde d'aluminium, mélangées à un liant tel qu'un silicate de sodium, un silicate d'éthyle, un sulfate d'ammonium, un phosphate d'aluminum, un phosphate de sodium, ou un phosphate acide d'ammonium.

Conformément à l'invention la masse de moulage du modèle de la dent doit présenter lors de sa solidification une légère expansion linéaire qui peut être par exemple de 0,1 % à 0,4 %.

Dans un mode de mise en oeuvre préféré de l'invention, l'oxyde métallique utilisé est constitué par une poudre d'alumine (Al$_2$O$_3$) comportant éventuellement en mélange une poudre d'oxyde de zirconium (ZrO$_2$) dans des proportions qui peuvent être importantes.

On peut également utiliser, conformément à l'invention, de l'oxyde de zirconium pur ou stabilisé à l'oxyde d'Yttrium.

La barbotine peut être obtenue selon l'invention, en plaçant par exemple 100 g de particules d'oxydes métalliques dans environ 12 à 20 g d'eau et en ajoutant entre 0,05 g et 0,5 g d'un agent stabilisant de la suspension qui peut être constitué par exemple par un alcool de polyvinyle, un acide acrylique, un ester cellulosique, ou du silicate de sodium.

Conformément à l'invention, il est également préférable de ramener le Ph de la barbotine à une valeur sensiblement neutre, par exemple à une valeur d'environ 7 à 8, à l'adjonction d'une substance convenable telle que acide citrique.

Avant d'utiliser la barbotine selon l'invention, il est préférable de procéder à un dégazage de cette dernière, en la soumettant à des ultrasons dans un récipient où l'on fait le vide.

Conformément à l'invention, la première cuisson de l'infrastructure constituée par la couche de particules d'oxydes métalliques appliquée sur le modèle de dent, a pour premier objectif de séparer ces deux éléments par rétraction de la masse de moulage qui constitue le modèle de la dent, ce qui dans le cas du plâtre est obtenu par perte d'au moins une partie de l'eau de constitution du gypse qui constitue le plâtre.

Une telle déshydratation du plâtre peut être obtenue en portant l'infrastructure de particules d'oxydes métalliques placée sur le modèle de la dent en plâtre, dans un four dont la température est élevée lentement, par exemple à raison de 1°C par minute, jusqu'à une température d'environ 180°C puis ultérieurement d'une manière qui peut être plus rapide jusqu'à environ 330°C.

Dans cette première phase du traitement thermique, le plâtre se sépare de l'infrastructure en poudre d'oxydes métalliques.

Conformément à l'invention, on doit ensuite réaliser un frittage léger des particules d'oxydes métalliques en phase solide, de manière à obtenir un squelette rigide, à porosité ouverte, qui subit lors du frittage un faible retrait qui est par exemple inférieur à 0,4 % et qui est compensé par l'expansion que subit le modèle de la dent lors de la solidification de la masse de moulage.

Conformément à l'invention, il est avantageux que ce frittage reste un frittage léger, qui a simplement pour effet de lier entre elles les particules d'oxydes métalliques sans provoquer un retrait notable du volume de l'infrastructure.

On obtient ainsi grâce à l'invention, des prothèses qui s'adaptent avec une très grande précision sur la dent naturelle.

Conformément à l'invention, le traitement thermique qui permet d'atteindre le résultat recherché, dépend à la fois de sa température, de la vitesse de montée en température, du temps pendant lequel cette température est maintenue et de la dimension moyenne des particules d'oxydes métalliques.

C'est ainsi par exemple, que pour un traitement thermique d'environ une à trois heures, la température peut être d'environ 1050°C à 1150°C avec des particules d'oxydes métalliques de dimension moyenne d'environ 3,5 microns.

La température de traitement peut être portée à 1250°C avec des particules d'oxydes métalliques de dimension moyenne d'environ 8,5 microns, et de 1300°C à 1400°C avec des particules d'oxydes métalliques de dimension moyenne d'environ 20 microns.

Une augmentation de la vitesse de montée en température a pour effet de réduire le retrait qui se produit lors du frittage.

Conformément à l'invention, il est préférable de ne pas utiliser des particules d'oxydes métalliques qui ont une surface spécifique trop élevée. On peut utiliser avantageusement selon l'invention des poudres d'oxydes métalliques ayant par exemple une surface spécifique d'environ 1 à 5 m$^2$ par g.

Conformément à l'invention, il est possible d'élever la température de frittage, tout en gardant un faible retrait, en mélangeant à la poudre d'oxyde d'aluminium et/ou d'oxyde de zirconium, d'autres poudres d'oxydes métalliques telles que de la poudre d'oxyde de magnésium (MgO). C'est ainsi qu'une addition d'environ 0,3 à 3 % de poudre d'oxyde de magnésium permet d'augmenter la température de frittage d'environ 150°C.

On peut également utiliser à cet effet une addition de poudre d'oxyde de lanthane (La$_2$O$_3$) ou encore des poudres d'autres oxydes tels que l'oxyde d'Yttrium (Y$_2$O$_3$) ou des oxydes de terres rares.

Le frittage des poudres d'oxydes métalliques à température relativement basse, par exemple à une température comprise entre 1050° et 1150°C, présente l'avantage de pouvoir travailler avec des fours analogues à ceux qui sont couramment utilisés dans l'art dentaire.

Un élèvement de la température de frittage des particules d'oxydes métalliques présente l'avantage de pouvoir réaliser l'imprégnation de l'infrastructure à l'aide du verre en opérant à une température plus élevée, ce qui permet une plus grande variété dans le choix du verre, étant entendu comme cela sera expliqué plus en détail ultérieurement, qu'il est indiqué conformément à l'invention, de réaliser l'imprégnation par le verre du squelette d'oxydes métalliques frittés sans augmenter le retrait résultant du frittage qui a été réalisé lors du premier traitement thermique. Ceci revient à dire que l'imprégnation avec le verre doit s'effectuer à une température au plus égale à la température du frittage et ceci pendant un temps relativement court dont il est préférable dans la pratique, qu'il ne dépasse pas 2 à 4 heures, mais qui dépend de l'épaisseur à imprégner.

Conformément à l'invention, le verre utilisé pour réaliser l'imprégnation du squelette constitué par la poudre d'oxydes métalliques frittés doit présenter un certain nombre de caractéristiques.

Bien que les pores du squelette métallique fritté selon l'invention puissent avoir des dimensions aussi faibles que 0,3 micron, il est préférable, conformément à l'invention, que le verre imprègne la totalité des pores de ce squelette.

Pour cela, il est préférable selon l'invention que le verre présente à la température de traitement, la particularité de mouiller le squelette d'oxydes métalliques frittés, ce qui revient à dire que l'énergie de surface du verre doit, à cette température, être inférieure à l'énergie de surface des particules d'oxydes métalliques.

Le caractère mouillant du verre peut être augmenté selon l'invention par introduction dans sa composition par exemple d'oxyde de bore, d'oxyde de plomb ou d'oxyde de vanadium.

Conformément à l'invention, le verre doit, de préférence, présenter à la température considérée, une faible viscosité, ce qui peut être obtenu par un choix approprié des proportions des divers oxydes, en augmentant par exemple la teneur en oxyde de bore, en oxyde de plomb ou en oxyde de lanthane.

Conformément à l'invention, la réactivité du verre vis-à-vis de l'oxyde métallique ne doit être ni trop forte, ni trop faible. Ceci est obtenu conformément à l'invention en utilisant un verre qui contient dès le départ les oxydes métalliques tels que Al$_2$O$_3$ et/ou ZrO$_2$ dans une teneur légèrement inférieure mais voisine de la saturation du verre vis-à-vis de ces oxydes métalliques à la température d'imprégnation.

Conformément à l'invention, le coefficient de dilatation du verre doit de préférence être inférieur, mais voisin du coefficient de dilatation du squelette de particules d'oxydes métalliques frittées, de manière à obtenir une bonne résistance de la prothèse aux chocs thermiques.

On peut agir sur le coefficient de dilatation du verre par exemple par l'adjonction d'oxyde de sodium, d'oxyde de potassium ou d'oxyde de lithium pour l'augmenter, ou d'oxyde de silicium ou d'oxyde de titane pour le diminuer.

Conformément à l'invention, le verre d'imprégnation peut contenir une faible quantité d'oxydes métalliques ou de métaux, par exemple jusqu'à 2 % pour donner une coloration à la prothèse et modifier ainsi la couleur du squelette en oxydes métalliques frittés.

Conformément à l'invention, en choisissant un verre d'imprégnation dont l'indice de réfraction est plus ou moins voisin de celui de l'infrastructure frittée, on obtient une prothèse plus ou moins translucide. On peut de la sorte faire varier l'indice du verre d'imprégnation pour obtenir des effets optiques différents.

Il convient par ailleurs, de souligner que, conformément à l'invention, le verre d'imprégnation est constitué principalement par un mélange d'oxydes qui après solidification peut se présenter à l'état complètement amorphe en étant transparent ou à l'état plus ou moins cristallisé en étant opalescent.

Pour réaliser l'imprégnation du squelette d'oxydes métalliques frittés à l'aide du verre, plusieurs techniques peuvent être utilisées conformément à l'invention.

On peut par exemple appliquer sur la surface externe de l'infrastructure de la prothèse (c'est-à-dire sur la surface de la prothèse, dont la forme n'a pas été déterminée par le modèle de la dent), une pâte de verre à l'eau, qui lorsqu'elle est portée à la température convenable, fond et diffuse spontanément à l'intérieur de tout le volume de l'infrastructure en comblant la totalité des pores.

On peut également, conformément à l'invention, disposer dans une coupelle, un lit de poudre de verre, sur lequel on pose l'infrastructure, puis on porte l'ensemble à la température désirée. Lorsque le verre est fondu, il occupe par capillarité la totalité du réseau de pores de l'infrastructure en particules d'oxydes métalliques frittées.

Conformément à l'invention, il est préférable que la surface de la prothèse qui doit venir au contact de la dent ne soit pas au contact du verre fondu et que le remplissage des pores situés au voisinage de cette surface s'effectue par capillarité à partir de l'intérieur de la masse de l'infrastructure d'oxydes métalliques frittés, de manière à ce qu'aucun excès de verre ne demeure sur cette surface, ce qui altérerait sa géométrie.

Conformément à l'invention, il est avantageux de recouvrir l'infrastructure constituée par les particules d'oxydes métalliques frittées imprégnées de verre par une ou plusieurs couches d'émail de propriétés optiques et de teintes différentes, de manière à donner à la prothèse l'apparence des dents naturelles.

Dans ce cas, il convient de modeler la masse de particules d'oxydes métalliques qui recouvre le modèle de la dent avant le frittage, pour lui donner une forme qui laisse subsister la place nécessaire à l'émaillage qui intervient à la fin du procédé selon l'invention.

Conformément à l'invention, il convient d'utiliser de préférence comme émail, un verre chargé qui présente un coefficient de dilatation légèrement inférieur à celui de l'infrastructure et dont la mise en forme s'effectue à partir d'une poudre mélangée à de l'eau qui est sculptée et dont la consolidation mécanique est obtenue par un frittage en phase liquide.

Conformément à l'invention, on peut utiliser avantageusement un émail de type boro-silicate alcalin contenant de l'alumine.

La présente invention a également pour objet, une prothèse dentaire obtenue par le procédé qui vient d'être décrit et qui est caractérisé par le fait qu'elle est constituée par une infrastructure (ou squelette) obtenue par frittage en phase solide de particules d'oxydes métalliques et constituant un réseau continu, dont les interstices sont occupés par du verre.

Dans le but de mieux faire comprendre l'invention, on va en décrire maintenant à titre d'illustration et sans aucun caractère limitatif, plusieurs modes de mise en oeuvre.

Exemple 1 : Réalisation d'une chape périphérique ou couronne.

On procède tout d'abord selon la technique habituelle à l'usinage de la dent et à la prise des empreintes qui permettent d'obtenir un premier modèle de travail qui restitue en positif la forme de la dent qui doit recevoir la couronne ainsi que celle des dents adjacentes et antagonistes.

On réalise ensuite conformément à l'invention un second modèle de la dent que doit recevoir la couronne dans un plâtre constitué par un mélange de 100 g d'hémi-hydrate de sulfate de calcium et de 21 cc d'eau.

Lorsque le plâtre représentant le modèle de la dent a fait sa prise et a séché, il a subi une expansion de 0,4 % environ. Il est ensuite immergé dans une barbotine conforme à l'invention qui est constituée par 100 g de poudre d'alumine d'une grosseur moyenne de grain de 3,5 microns mélangée à 13 cc d'eau contenant 0,5 g d'ester cellulosique, le pH étant ramené à 7,6 par adjonction de 0,07 g d'acide citrique.

L'absorption de l'eau par les capillaires du plâtre provoque une agglomération des particules d'alumine à la surface du modèle, selon une épaisseur qui est fonction du temps d'immersion dans la barbotine, et qui peut être par exemple de l'ordre de 0,5 à 1 mm ou plus si cela est nécessaire.

Le technicien de laboratoire peut modifier l'épaisseur du dépôt d'alumine, soit à l'aide d'un pinceau en apportant de la barbotine aux endroits à surépaissir, soit à l'aide d'une spatule grâce à laquelle il peut éliminer un excès de dépôt et finir les bords.

Il est ainsi possible de procéder facilement à la sculpture de la prothèse dès que le dépôt d'alumine a sensiblement la consistance de la terre glaise.

Le technicien place ensuite le modèle en plâtre qui supporte la couche d'alumine ainsi réalisée, dans une étuve, de préférence sous vide, pour faciliter le sèchage, à moins qu'il ne préfère laisser sécher à l'air.

L'ensemble est ensuite placé dans un four où il est porté dans un premier temps à une température d'environ 180°C en élevant la température d'environ 1°C par minute, puis à une température d'environ 330°C, la durée totale de l'opération pouvant s'étager sur trois à cinq heures par exemple.

On obtient de cette manière une élimination de l'eau de constitution du plâtre et une séparation du modèle et de l'infrastructure en alumine.

Sans sortir du four, on élève alors progressivement la température par exemple en environ 1 heure, à environ 1100°C, température que l'on maintient pendant environ deux heures.

Après avoir laissé refroidir, on constate que le modèle en plâtre s'est rétracté fortement, et que l'infrastructure en particules d'alumine frittées est suffisamment consolidée pour pouvoir être manipulée.

Le frittage ainsi réalisé s'effectue avec un retrait de 0,3 % qui est compensé par l'expansion du plâtre du modèle de la dent lors de sa prise, en tenant compte d'un jeu de 0,1 % pour la mise en place de la prothèse.

Pour effectuer l'imprégnation conforme à l'invention à l'aide du verre à l'état fondu, on place l'infrastructure en alumine frittée dans une coupelle sur un lit de poudre de verre dont la composition en poids est la suivante :

$$
\begin{array}{ll}
\text{Silice } (SiO_2) \dots\dots\dots\dots\dots\dots & 20 \text{ g} \\
\text{Oxyde Bore } (B_2O_3) \dots\dots\dots\dots\dots & 19 \text{ g} \\
\text{Oxyde d'Aluminium } (Al_2O_3) \dots\dots & 20 \text{ g} \\
\text{Oxyde de Lanthane } (La_2O_3) \dots\dots & 30 \text{ g} \\
\text{Oxyde de Calcium } (CaO) \dots\dots\dots & 5 \text{ g} \\
\text{Oxyde de Titane } (TiO_2) \dots\dots\dots & 4 \text{ g} \\
\text{Oxydes colorants } \dots\dots\dots\dots\dots & 2 \text{ g}
\end{array}
$$

On porte progressivement à une température légèrement inférieure à 1100°C que l'on maintient pendant deux à trois heures, pour permettre au verre fondu de pénétrer par capillarité dans l'infrastructure en particules d'alumine, de manière à occuper la totalité des pores qu'elle comporte.

Selon l'invention, la couronne ainsi préfabriquée est ensuite émaillée par application de plusieurs couches d'émail dont la composition en poids du verre est la suivante :

$$
\begin{array}{ll}
\text{Oxyde de Sodium } (Na_2O) \dots\dots\dots & 4,6 \text{ g} \\
\text{Oxyde de Potassium } (K_2O) \dots\dots\dots & 7,6 \text{ g} \\
\text{Oxyde de Calcium } (CaO) \dots\dots\dots & 1,7 \text{ g} \\
\text{Oxyde d'Aluminium } (Al_2O_3) \dots\dots & 13,9 \text{ g} \\
\text{Oxyde de Silicium } (SiO_2) \dots\dots\dots & 65,5 \text{ g} \\
\text{Oxyde de Bore } (B_2O_3) \dots\dots\dots\dots & 6,7 \text{ g}
\end{array}
$$

Des additifs de coloration et les charges telles que l'oxyde d'étain, l'oxyde de Silicium (quartz), l'oxyde de Zirconium ou l'oxyde d'aluminium variant avec les couches successives.

Lorsque la prothèse selon l'invention n'est pas destinée à être émaillée, il est clair que l'on doit donner au dépôt de particules d'alumine sur le modèle de dent en plâtre, une forme qui correspond à la forme extérieure que l'on désire conférer à la prothèse. Celle-ci est obtenue en modelant ou sculptant le dépôt de particules d'alumine selon une technique habituelle pour les prothèses dentaires.

Exemple 2 : Réalisation d'une chape périphérique ou couronne.

On procède comme indiqué à l'exemple 1, à la différence près que le second modèle de la dent qui doit recevoir la couronne est réalisé avec un mélange ayant la composition suivante :

```
Charge réfractaire de Silice (SiO₂)
ou d'alumine (Al₂O₃) ................... 75   g
Oxyde de Magnésium (MgO) .............. 10   g
Phosphate acide d'ammonium (NH₄ H₂ PO₄). 15   g
Eau ................................... 24   g
```

La barbotine est constituée par un mélange ayant la composition suivante :

```
Oxyde d'Aluminium (Al₂O₃) ............. 97   g
Oxyde de Magnésium (MgO) .............. 3    g
Eau ................................... 17   g
Alcool polyvinylique .................. 0,25 g
```

Les oxydes métalliques ont une dimension moyenne de grain de 8 microns. Le pH de la composition est amené à 8 par addition d'acide picrique.

Le frittage est obtenu en portant le modèle de dent revêtu de la couche d'oxydes métalliques à la température de 1250°C pendant 1 heure.

L'imprégnation de l'infrastructure ainsi obtenue est réalisée en la mettant en contact pendant deux heures à la température de 1200°C avec un verre ayant la composition suivante :

```
Silice (SiO₂) ......................... 10   g
Oxyde de Bore (B₂O₃) .................. 12,5 g
Alumine (Al₂O₃)........................ 20   g
Oxyde d'Yttrium (Y₂O₃) ................ 20   g
Oxyde de Lanthane (La₂O₃) ............. 25   g
Oxyde de Titane (TiO₂) ................ 5    g
Oxyde de Calcium (CaO) ................ 5    g
Oxyde de Cérium (CeO) ................. 2,5  g
```

7

Exemple 3 : Réalisation d'une chape périphérique ou couronne.

On procède comme indiqué à l'exemple 1 en réalisant le second modèle de la dent à l'aide de :

$$
\begin{aligned}
&\text{Charge réfractaire de Silice } (SiO_2) \\
&\text{ou d'alumine } (Al_2O_3) \ldots\ldots\ldots\ldots\ldots\ldots\ 75 \text{ g} \\
&\text{Oxyde de Magnésium } (MgO) \ldots\ldots\ldots\ldots\ 10 \text{ g} \\
&\text{Phosphate acide d'ammonium } (NH_4\ H_2\ PO_4).\ 15 \text{ g} \\
&\text{Eau } \ldots\ldots\ldots\ldots\ldots\ldots\ldots\ldots\ldots\ldots\ 24 \text{ g}
\end{aligned}
$$

On immerge ce modèle dans une barbotine ayant la composition suivante :

$$
\begin{aligned}
&\text{Alumine } (AlO_3) \ldots\ldots\ldots\ldots\ldots\ldots\ldots\ 300 \quad \text{g} \\
&\text{Eau } \ldots\ldots\ldots\ldots\ldots\ldots\ldots\ldots\ldots\ 20 \quad \text{g} \\
&\text{Silicate de Sodium } \ldots\ldots\ldots\ldots\ldots\ 0,5 \text{ g}
\end{aligned}
$$

Les particules d'alumine ont une dimension moyenne de grains de 20 microns.
Le frittage est réalisé à la température de 1300°C pendant environ 2 heures.
L'imprégnation est réalisée avec un verre ayant la composition suivante :

$$
\begin{aligned}
&\text{Oxyde de Silicium } (SiO_2) \ldots\ldots\ldots\ldots\ 63 \text{ g} \\
&\text{Oxyde d'Aluminium } (Al_2O_3) \ldots\ldots\ldots\ldots\ 14 \text{ g} \\
&\text{Oxyde de Calcium } (CaO) \ldots\ldots\ldots\ldots\ 23 \text{ g}
\end{aligned}
$$

Exemple 4 : Réalisation d'une chape périphérique ou couronne.

On procède comme indiqué à l'exemple 1 en plongeant le modèle de la dent en plâtre dans une barbotine ayant la constitution suivante :

$$
\begin{aligned}
&\text{Alumine } (Al_2O_3) \ldots\ldots\ldots\ldots\ldots\ldots\ 80 \text{ g} \\
&\text{Oxyde de Zirconium } (ZrO_2) \\
&\text{Stabilisé à l'Yttrium}\ldots\ldots\ldots\ldots\ldots\ 20 \text{ g} \\
&\text{Eau } \ldots\ldots\ldots\ldots\ldots\ldots\ldots\ldots\ldots\ 15 \text{ g} \\
&\text{Alcool de polyvinyle } \ldots\ldots\ldots\ldots\ 0,1 \text{ g}
\end{aligned}
$$

Les particules d'alumine ont une dimension moyenne de grains d'environ 3,5 microns
Les particules d'oxyde de Zirconium ont une dimension moyenne de grains de 0,5 microns.
Le pH est amené à la valeur 7 par adjonction d'acide citrique.
Le frittage est réalisé pendant trois heures à la température de 1150°C.

EP 0 241 384 B1

L'imprégnation de l'infrastructure frittée s'effectue avec un verre ayant la composition suivante :

$$
\begin{array}{llll}
\text{Silice (SiO}_2) \ldots\ldots\ldots\ldots & 22 & \text{g} \\
\text{Oxyde de Bore (B}_2\text{O}_3) \ldots\ldots\ldots & 15 & \text{g} \\
\text{Alumine (Al}_2\text{O}_3) \ldots\ldots\ldots\ldots & 20 & \text{g} \\
\text{Oxyde de Zirconium (ZrO}_2) \ldots\ldots & 2 & \text{g} \\
\text{Oxyde de Lanthane (La}_2\text{O}_3) \ldots\ldots & 28 & \text{g} \\
\text{Oxyde de Calcium (CaO)} \ldots\ldots\ldots & 7 & \text{g} \\
\text{Oxyde de Titane (TiO}_2) \ldots\ldots\ldots & 3 & \text{g} \\
\text{Oxyde de Fer (Fe}_2\text{O}_3) \ldots\ldots\ldots & 2 & \text{g} \\
\text{Oxyde de Cérium (CeO)} \ldots\ldots\ldots & 1 & \text{g}
\end{array}
$$

Exemple 5 : Réalisation d'une prothèse intéressant simultanément plusieurs dents.

Pour réaliser une prothèse intéressant simultanément plusieurs dents telle qu'un "bridge", on opère comme indiqué aux exemples 1 à 4 mais en utilisant un modèle qui comprend les différentes dents qui doivent recevoir la prothèse et en sculptant le dépôt de particules d'oxydes métalliques en fonction de la forme que l'on désire donner à la prothèse.

Dans les exemples 2 à 5, on peut selon l'invention, procéder à un émaillage par couches successives à l'aide de verres ayant une composition en poids du type suivant :

$$
\begin{array}{lll}
\text{Oxyde de Sodium (Na}_2\text{O)} \ldots\ldots\ldots & 4,7 \text{ à } 4,2 \text{ g} \\
\text{Oxyde de Potassium (K}_2\text{O)} \ldots\ldots & 8,2 \text{ à } 6,8 \text{ g} \\
\text{Oxyde de Calcium (CaO)} \ldots\ldots\ldots & 1,8 \text{ à } 1,5 \text{ g} \\
\text{Oxyde d'Aluminium (Al}_2\text{O}_3) \ldots\ldots & 15 \text{ à } 13 \text{ g} \\
\text{Oxyde de Silicium (SiO}_2) \ldots\ldots & 62,8 \text{ à } 68 \text{ g} \\
\text{Oxyde de Bore (B}_2\text{O}_3) \ldots\ldots\ldots & 7,5 \text{ à } 6,5 \text{ g}
\end{array}
$$

Exemple 6 : Réalisation d'une pièce de reconstitution partielle.

Ces pièces qui sont généralement connues sous les noms de "inlay" et "onlay" servent à remplir une cavité qui est réalisée avec dépouille dans la dent.

Pour cela, on prend une empreinte qui permet de reconstituer un modèle positif de la dent de plâtre ou d'un autre matériau poreux et l'on dépose, par exemple à l'aide d'un pinceau, la barbotine dans la cavité du modèle de manière à garnir cette dernière avec une quantité suffisante de particules d'oxydes métalliques que l'on sculpte pour reconstituer la partie manquante de la dent.

On procède ensuite comme indiqué aux exemples précédents, étant entendu que dans ce cas on n'a généralement pas à revêtir la prothèse obtenue d'une couche d'émail.

**Revendications**

1. Procédé pour fabriquer des prothèses dentaires céramiques, caractérisé par le fait que l'on réalise un modèle de la dent qui doit recevoir la prothèse dans une masse de moulage qui présente lors de sa solidification une légère expansion linéaire ; que l'on prépare une barbotine constituée par une suspension dans l'eau de particules d'oxydes métalliques additionnée d'un agent de stabilisation de la suspension et éventuellement d'un agent du contrôle du Ph ; que l'on met en contact le modèle de la dent avec la barbotine, de telle sorte que les particules d'oxydes métalliques s'agglomèrent sur la

9

surface du modèle de la dent jusqu'à ce que l'on obtienne une épaisseur suffisante ; que l'on donne à la couche d'oxydes métalliques la forme extérieure que l'on désire conférer à l'infrastructure de la prothèse ; que l'on procède à la cuisson du modèle de la dent ainsi revêtu de l'infrastructure à base d'oxydes métalliques pour réaliser dans un premier temps une déshydratation du modèle de la dent qui provoque son retrait, puis un léger frittage en phase solide des particules d'oxydes métalliques ; et que l'on imprègne d'un verre, l'infrastructure ainsi frittée à une température suffisante pour qu'il occupe la totalité des pores ouverts que l'infrastructure comporte.

2. Procédé selon la revendication 1, caractérisé par le fait que l'on procède ensuite au revêtement de la surface externe de la prothèse avec un émail compatible avec la nature du verre ayant servi à l'imprégnation de l'infrastructure et possédant un coefficient de dilatation voisin et de préférence inférieur à celui de cette dernière.

3. Procédé selon la revendication 2, caractérisé par le fait que l'émail est constitué par un verre de type boro-silicate alcalin contenant de l'alumine.

4. Procédé selon l'une quelconque des revendications précédentes, caractérisé par le fait que l'on réalise le moulage du modèle de la dent à l'aide d'une masse de moulage qui, lors de sa solidification, présente une expansion linéaire comprise entre environ 0,1 % et environ 0,4 %.

5. Procédé selon la revendication 4, caractérisé par le fait que la masse de moulage est un plâtre constitué par un hémi-hydrate de sulfate de calcium ne comportant aucune charge minérale.

6. Procédé selon la revendication 4, caractérisé par le fait que la masse de moulage est constituée par une charge réfractaire telle que l'oxyde de Silicium ou l'oxyde d'Aluminium, mélangée à un liant tel que le silicate de sodium, le silicate d'éthyle, le sulfate d'ammonium, le phosphate d'aluminium, ou le phosphate de sodium.

7. Procédé selon l'une quelconque des revendications précédentes, caractérisé par le fait que la poudre d'oxydes métalliques utilisée, est constituée par des particules d'alumine ($Al_2O_3$) comportant éventuellement en mélange des particules d'oxyde de zirconium ($ZrO_2$).

8. Procédé selon l'une quelconque des revendications précédentes, caractérisé par le fait que l'on utilise une barbotine comportant entre environ 0,05 % et 0,5 % en poids par rapport aux oxydes métalliques d'un agent stabilisant de la suspension tel qu'un alcool de polyvinyle, un acide acrylique, un ester cellulosique ou du silicate de sodium.

9. Procédé selon l'une quelconque des revendications précédentes, caractérisé par le fait que l'on amène le Ph de la barbotine à une valeur comprise entre environ 7 et environ 8 par l'adjonction d'une substance telle que de l'acide citrique.

10. Procédé selon l'une quelconque des revendications précédentes, caractérisé par le fait qu'avant de l'appliquer sur le modèle de la dent, on procède à un dégazage de la barbotine en la soumettant à des ultrasons dans un récipient où l'on fait le vide.

11. Procédé selon l'une quelconque des revendications précédentes, caractérisé par le fait qu'au début de la cuisson du modèle de la dent supportant la couche de particules d'oxydes métalliques, on élève la température lentement, par exemple en raison de 1°C par minute, jusqu'à une température d'environ 350°C et qu'après quoi, on amène la température à une valeur au moins égale à environ 1050°C pendant un temps suffisant pour provoquer un léger frittage en phase solide des particules d'oxydes métalliques.

12. Procédé selon la revendication 11, caractérisé par le fait que lors du frittage des particules d'oxydes métalliques, l'infrastructure subit un faible retrait, de préférence inférieur à 0,4 %, qui correspond à l'expansion qu'a subie le modèle de la dent lors de la solidification de la masse de moulage.

13. Procédé selon l'une quelconque des revendications précédentes, caractérisé par le fait que la température de frittage est d'environ de 1050°C à 1150°C pour des particules d'alumine dont la dimension moyenne est d'environ 3,5 microns.

14. Procédé selon l'une quelconque des revendications 1 à 12, caractérisé par le fait que le traitement thermique est effectué à une température d'environ 1250°C avec des particules d'alumine dont la dimension moyenne est d'environ 8,5 microns.

15. Procédé selon l'une quelconque des revendications 1 à 12, caractérisé par le fait que le traitement thermique est effectué à une température d'environ 1300°C à 1400°C avec des particules d'alumine dont la dimension moyenne est d'environ 20 microns.

16. Procédé selon l'une quelconque des revendications précédentes, caractérisé par le fait que l'on utilise des particules d'oxydes métalliques ayant une surface spécifique comprise entre environ 1 et 5 m2 par gramme.

17. Procédé selon l'une quelconque des revendications précédentes, caractérisé par le fait que l'on procède au frittage à une température plus élevée en mélangeant à la poudre d'oxyde d'aluminium et/ou d'oxyde de zirconium, d'autres poudres d'oxydes métalliques tels que l'oxyde de magnésium (MgO), l'oxyde de lantane ($La_2O_3$) ou des oxydes de terres rares.

18. Procédé selon l'une quelconque des revendications précédentes, caractérisé par le fait que l'imprégnation de l'infrastructure en particules d'oxydes métalliques frittés, est réalisée à l'aide d'un verre qui possède une énergie de surface à la température de traitement, qui est inférieure à l'énergie de surface des particules d'oxydes métalliques.

19. Procédé selon l'une quelconque des revendications précédentes, caractérisé par le fait que l'on augmente le caractère mouillant du verre d'imprégnation en augmentant sa teneur en un constituant tel que l'oxyde de plomb, l'oxyde de bore ou l'oxyde de vanadium.

20. Procédé selon l'une quelconque des revendications précédentes, caractérisé par le fait que l'on utilise pour imprégner l'infrastructure en particules d'oxydes métalliques frittés, un verre qui contient dans sa composition les mêmes oxydes métalliques que ceux constituant l'infrastructure et ceci dans une teneur légèrement inférieure mais voisine de la saturation du verre vis-à-vis de ces oxydes métalliques à la température d'imprégnation.

21. Procédé selon l'une quelconque des revendications précédentes, caractérisé par le fait que l'on réalise l'imprégnation de l'infrastructure en particules d'oxydes métalliques frittés à l'aide d'un verre qui possède un coefficient de dilatation inférieur mais voisin de celui de l'infrastructure.

22. Procédé selon l'une quelconque des revendications précédentes, caractérisé par le fait que l'on réalise l'imprégnation de l'infrastructure en particules d'oxydes métalliques frittés à l'aide d'un verre contenant par exemple jusqu'à 2 % d'oxydes métalliques ou de métaux pour donner une coloration à la prothèse et modifier ainsi la couleur du squelette en oxydes métalliques frittés.

23. Procédé selon l'une quelconque des revendications précédentes, caractérisé par le fait que l'on réalise l'imprégnation de l'infrastructure en particules d'oxydes métalliques frittés à l'aide d'un verre constitué principalement par un mélange d'oxydes qui après solidification se présente à l'état totalement amorphe en étant transparent ou à l'état plus ou moins cristallisé en étant opalescent.

24. Procédé selon l'une quelconque des revendications précédentes, caractérisé par le fait que l'imprégnation de l'infrastructure d'oxydes métalliques frittés au voisinage des surfaces devant venir en contact avec la dent naturelle s'effectue grâce à la diffusion capillaire du verre par l'intérieur de l'infrastructure en particules d'oxydes métalliques frittés.

25. Procédé selon l'une quelconque des revendications précédentes, caractérisé par le fait que l'on détermine l'opacité de la prothèse obtenue en utilisant pour l'imprégnation de l'infrastructure un verre dont l'indice de réfraction est plus ou moins différent de l'indice de réfraction de l'infrastructure.

**26.** Prothèse dentaire céramique, caractérisée par le fait qu'elle est constituée par une infrastructure continue rigide, à pores ouverts, obtenue par frittage en phase solide de particules d'oxydes métalliques et dont les interstices sont occupés par du verre.

**27.** Prothèse selon la revendication 26, caractérisée par le fait qu'elle est revêtue d'un émail constitué par un verre de type boro-silicate alcalin contenant de l'alumine.

**Claims**

**1.** Process for manufacturing ceramic dental prostheses, characterised by the fact that a model of the tooth which is to receive the prosthesis is made in a casting composition which exhibits, upon its solidification, a slight linear expansion; that a slip is prepared which consists of a water-suspension of metallic oxide particles to which is added an agent for stabilising the suspension and, optionally, an agent for controlling the pH; that the model of the tooth is brought into contact with the slip, so that the metallic oxide particles agglomerate on the surface of the model of the tooth until a sufficient thickness is obtained; that the layer of metallic oxides is given the outer shape which it is desired to confer upon the infrastructure of the prosthesis; that the model of the tooth thus coated with the metallic oxide-based infrastructure is baked in order to effect, in a first stage, a dehydration of the model of the tooth which causes its shrinkage, then a slight solid-phase sintering of the metallic oxide particles; and that a glass is used to impregnate the infrastructure thus sintered at a temperature sufficient for it to occupy all of the open pores which the infrastructure comprises.

**2.** Process according to Claim 1, characterised by the fact that the outer surface of the prosthesis is then coated with an enamel compatible with the nature of the glass used for the impregnation of the infrastructure and having a coefficient of expansion close to and preferably below that of the latter.

**3.** Process according to Claim 2, characterised by the fact that the enamel consists of a glass of the alkali metal borosilicate type containing alumina.

**4.** Process according to any one of the preceding claims, characterised by the fact that the casting of the model of the tooth is carried out using a casting composition which, upon its solidification, exhibits a linear expansion of between approximately 0.1% and approximately 0.4%.

**5.** Process according to Claim 4, characterised by the fact that the casting composition is a plaster consisting of a calcium sulphate hemihydrate comprising no mineral charge.

**6.** Process according to Claim 4, characterised by the fact that the casting composition consists of a refractory charge such as silicon dioxide or aluminium oxide, mixed with a binder such as sodium silicate, ethyl silicate, ammonium sulphate, aluminium phosphate or sodium phosphate.

**7.** Process according to any one of the preceding claims, characterised by the fact that the powder of metallic oxides used consists of particles of alumina ($Al_2O_3$) optionally comprising in a mixture particles of zirconium oxide ($ZrO_2$).

**8.** Process according to any one of the preceding claims, characterised by the fact that a slip is used which comprises between approximately 0.05% and 0.5% by weight, relative to the metallic oxides, of an agent stabilising the suspension, such as a polyvinyl alcohol, an acrylic acid, a cellulose ester or sodium silicate.

**9.** Process according to any one of the preceding claims, characterised by the fact that the pH of the slip is brought to a value of between approximately 7 and approximately 8 by adding a substance such as citric acid.

**10.** Process according to any one of the preceding claims, characterised by the fact that, before applying it to the model of the tooth, the slip is degassed by subjecting it to ultrasonics in a container in which a vacuum is established.

EP 0 241 384 B1

11. Process according to any one of the preceding claims, characterised by the fact that, at the start of the baking of the model of the tooth supporting the layer of metallic oxide particles, the temperature is raised slowly, for example at a rate of 1°C per minute, up to a temperature of approximately 350°C and that, after this, the temperature is brought to a value at least equal to approximately 1050°C for a period sufficient to cause a slight solid-phase sintering of the metallic oxide particles.

12. Process according to Claim 11, characterised by the fact that, during the sintering of the metallic oxide particles, the infrastructure undergoes a slight shrinkage, preferably less than 0.4%, which corresponds to the expansion which the model of the tooth has undergone during the solidification of the casting composition.

13. Process according to any one of the preceding claims, characterised by the fact that the sintering temperature is approximately 1050°C to 1150°C for alumina particles whose average size is approximately 3.5 microns.

14. Process according to any one of Claims 1 to 12, characterised by the fact that the thermal treatment is carried out at a temperature of approximately 1250°C with alumina particles whose average size is approximately 8.5 microns.

15. Process according to any one of Claims 1 to 12, characterised by the fact that the thermal treatment is carried out at a temperature of approximately 1300°C to 1400°C with alumina particles whose average size is approximately 20 microns.

16. Process according to any one of the preceding claims, characterised by the fact that metallic oxide particles are used which have a specific surface area of between approximately 1 and 5 $m^2$ per gram.

17. Process according to any one of the preceding claims, characterised by the fact that the sintering is carried out at a higher temperature by mixing with the aluminium oxide and/or zirconium oxide powder other powders of metallic oxides, such as magnesium oxide (MgO), lanthanum oxide ($La_2O_3$) or rare earth oxides.

18. Process according to any one of the preceding claims, characterised by the fact that the impregnation of the infrastructure of sintered metallic oxide particles is carried out using a glass which has a surface energy at the treatment temperature which is lower than the surface energy of the metallic oxide particles.

19. Process according to any one of the preceding claims, characterised by the fact that the wetting character of the impregnation glass is increased by increasing the content therein of a constituent such as lead oxide, boron oxide or vanadium oxide.

20. Process according to any one of the preceding claims, characterised by the fact that, in order to impregnate the infrastructure of sintered metallic oxide particles, a glass is used which contains in its composition the same metallic oxides as those constituting the infrastructure, and this in a content which is slightly lower than but close to the saturation of the glass with respect to these metallic oxides at the impregnation temperature.

21. Process according to any one of the preceding claims, characterised by the fact that the impregnation of the infrastructure of sintered metallic oxide particles is carried out using a glass which has a coefficient of expansion lower than but close to that of the infrastructure.

22. Process according to any one of the preceding claims, characterised by the fact the impregnation of the infrastructure of sintered metallic oxide particles is carried out using a glass containing, for example, up to 2% of metallic oxides or metals in order to give the prosthesis a coloration and thus to modify the colour of the sintered metallic oxide skeleton.

23. Process according to any one of the preceding claims, characterised by the fact that the impregnation of the infrastructure of sintered metallic oxide particles is carried out using a glass consisting principally of a mixture of oxides which, after solidification, is in the completely amorphous state, being transparent, or in the more or less crystallised state, being opalescent.

24. Process according to any one of the preceding claims, characterised by the fact that the impregnation of the infrastructure of sintered metallic oxides in the vicinity of the surfaces which are to come into contact with the natural tooth is effected by virtue of the capillary diffusion of the glass through the inside of the infrastructure of sintered metallic oxide particles.

25. Process according to any one of the preceding claims, characterised by the fact that the opacity of the prosthesis obtained is determined by using, for the impregnation of the infrastructure, a glass whose refractive index is to a greater or lesser extent different from the refractive index of the infrastructure.

26. Ceramic dental prosthesis, characterised by the fact that it consists of a continuous, rigid, open-pore infrastructure obtained by solid-phase sintering of metallic oxide particles and whose interstices are occupied by glass.

27. Prosthesis according to Claim 26, characterised by the fact that it is coated with an enamel consisting of a glass of the alkali metal borosilicate type containing alumina.

**Patentansprüche**

1. Verfahren zur Herstellung von Zahnprothesen aus Keramik, dadurch gekennzeichnet, daß ein Modell des Zahns, der die Prothese erhalten soll, mit einer Formmasse, die sich während der Härtung geringfügig linear ausdehnt, hergestellt wird; daß eine Aufschlämmung, bestehend aus einer wäßrigen Suspension von Metalloxidpartikeln, der ein Suspensionsstabilisator sowie gegebenenfalls ein Mittel zur Kontrolle des pH-Werts zugesetzt ist, hergestellt wird; daß das Modell des Zahns derart mit der Aufschlämmung in Berührung gebracht wird, daß sich die Metalloxidpartikel auf der Oberfläche des Zahnmodells ansammeln, bis eine ausreichende Dicke erreicht ist; daß der Metalloxidschicht die äußere Form gegeben wird, die dem Unterbau der Prothese verliehen werden soll; daß das Brennen des Zahnmodells, das auf diese Weise mit dem Unterbau auf Metalloxidbasis beschichtet ist, vorgenommen wird, um in einem ersten Schritt eine Dehydratisierung des Zahnmodells durchzuführen, die das Schrumpfen des Modells bewirkt, und daß dann eine leichte Sinterung in der festen Phase der Metalloxidpartikel erfolgt; und daß der auf diese Weise gesinterte Unterbau bei einer Temperatur mit einem Glas imprägniert wird, die ausreicht, daß alle offenen Poren, die der Unterbau besitzt, mit dem Glas gefüllt werden.

2. Verfahren nach Anspruch 1, dadurch gekennzeichnet, daß anschließend die Beschichtung der äußeren Prothesenoberfläche mit einer Emaille, die mit der Natur des Glases, das zum Imprägnieren des Unterbaus diente, verträglich ist, und die einen ähnlichen und vorzugsweise einen geringeren Ausdehnungskoeffizienten besitzt als das Glas, vorgenommen wird.

3. Verfahren nach Anspruch 2, dadurch gekennzeichnet, daß die Emaille aus einem alkalischen Borosilikatglas, das Aluminiumoxid enthält, besteht.

4. Verfahren nach einem der vorhergehenden Ansprüche, dadurch gekennzeichnet, daß das Formen des Zahnmodells mittels einer Formmasse erfolgt, die während der Härtung eine lineare Ausdehnung von etwa 0,1 bis etwa 0,4 % zeigt.

5. Verfahren nach Anspruch 4, dadurch gekennzeichnet, daß die Formmasse ein Gips ist, der aus einem keine mineralischen Füllstoffe enthaltenden Calciumsulfathalbhydrat besteht.

6. Verfahren nach Anspruch 4, dadurch gekennzeichnet, daß die Formmasse aus einem feuerfesten Material wie Siliciumoxid oder Aluminiumoxid in Mischung mit einem Bindemittel wie Natriumsilicat, Ethylsilicat, Ammoniumsulfat, Aluminiumphosphat oder Natriumphosphat besteht.

7. Verfahren nach einem der vorhergehenden Ansprüche, dadurch gekennzeichnet, daß das verwendete Metalloxidpulver aus Aluminiumoxidpartikeln ($Al_2O_3$) besteht, die gegebenenfalls in Mischung mit Zirkoniumoxidpartikeln ($ZrO_2$) vorliegen.

8. Verfahren nach einem der vorhergehenden Ansprüche, dadurch gekennzeichnet, daß eine Aufschlämmung verwendet wird, die, bezogen auf die Metalloxide, zwischen ungefähr 0,05 und 0,5 Gew.-% eines die Suspension stabilisierenden Mittels, wie eines Polyvinylalkohols, einer Acrylsäure, eines Celluloseesters oder eines Natriumsilikats, enthält.

9. Verfahren nach einem der vorhergehenden Ansprüche, dadurch gekennzeichnet, daß der pH-Wert der Keramik durch Zusatz einer Substanz, wie Zitronensäure, auf einen Wert zwischen etwa 7 und etwa 8 eingestellt wird.

10. Verfahren nach einem der vorhergehenden Ansprüche, dadurch gekennzeichnet, daß die Aufschlämmung, ehe sie auf das Zahnmodell aufgetragen wird, entgast wird, indem sie in einem unter Vakuum gehaltenen Behälter mit Ultraschall behandelt wird.

11. Verfahren nach einem der vorhergehenden Ansprüche, dadurch gekennzeichnet, daß zu Beginn des Brennens des Zahnmodells, das die Schicht aus den Metalloxidpartikeln trägt, die Temperatur langsam erhöht wird, z.B. um 1°C pro Minute, bis zu einer Temperatur von etwa 350°C, und daß dann die Temperatur für eine Zeit, die ausreichend ist, um eine leichte Sinterung in der festen Phase der Metalloxidpartikel zu bewirken, auf einen Wert von mindestens 1050°C eingestellt wird.

12. Verfahren nach Anspruch 11, dadurch gekennzeichnet, daß der Unterbau während der Sinterung der Metalloxidpartikel geringfügig schrumpft, vorzugsweise um weniger als 0,4 %, was der Ausdehnung, die das Zahnmodell während der Härtung der Formmasse erfährt, entspricht.

13. Verfahren nach einem der vorangehenden Ansprüche, dadurch gekennzeichnet, daß die Sinterungstemperatur für Aluminiumoxidpartikel, deren mittlere Größe etwa 3,5 $\mu$m beträgt, ungefähr 1050°C bis 1150°C ist.

14. Verfahren nach einem der Ansprüche 1 bis 12, dadurch gekennzeichnet, daß die thermische Behandlung von Aluminiumoxidpartikeln, deren mittlere Größe etwa 8,5 $\mu$m beträgt, bei einer Temperatur von etwa 1250°C durchgeführt wird.

15. Verfahren nach einem der Ansprüche 1 bis 12, dadurch gekennzeichnet, daß die thermische Behandlung von Aluminiumoxidpartikeln, deren mittlere Größe etwa 20 $\mu$m beträgt, bei einer Temperatur von etwa 1300°C bis 1400°C durchgeführt wird.

16. Verfahren nach einem der vorangehenden Ansprüche, dadurch gekennzeichnet, daß Metalloxidpartikel mit einer spezifischen Oberfläche von etwa 1 bis 5 m$^2$/g verwendet werden.

17. Verfahren nach einem der vorangehenden Ansprüche, dadurch gekennzeichnet, daß die Sinterung bei einer noch höheren Temperatur erfolgt, indem dem Pulver aus Aluminiumoxid und/oder Zirkoniumoxid andere Metalloxidpulver wie Magnesiumoxid (MgO), Lanthanoxid (La$_2$O$_3$) oder Seltene Erdoxide beigemischt werden.

18. Verfahren nach einem der vorangehenden Ansprüche, dadurch gekennzeichnet, daß die Imprägnierung des Unterbaus aus gesinterten Metalloxidpartikeln mittels eines Glases durchgeführt wird, das bei der Behandlungstemperatur eine Oberflächenenergie aufweist, die unterhalb der Oberflächenenergie der Metalloxidpartikel liegt.

19. Verfahren nach einem der vorhergehenden Ansprüche, dadurch gekennzeichnet, daß die Benetzungsfähigkeit des zur Imprägnierung verwendeten Glases durch Erhöhung seines Gehalts an einem Bestandteil wie Bleioxid, Boroxid oder Vanadiumoxid erhöht wird.

20. Verfahren nach einem der vorhergehenden Ansprüche, dadurch gekennzeichnet, daß zur Imprägnierung des Unterbaus aus gesinterten Metalloxidpartikeln ein Glas verwendet wird, das in seiner Zusammensetzung dieselben Metalloxide enthält wie die, die den Unterbau bilden, und daß diese in einer Menge, die geringfügig unterhalb, jedoch nahe dem Sättigungswert des Glases bezüglich dieser Metalloxide bei der Imprägnierungstemperatur liegt, verwendet werden.

21. Verfahren nach einem der vorhergehenden Ansprüche, dadurch gekennzeichnet, daß die Imprägnierung des Unterbaus aus gesinterten Metalloxidpartikeln mittels eines Glases erfolgt, dessen Ausdehnungskoeffizient niedriger als der des Unterbaus ist, aber in dessen Nähe liegt.

22. Verfahren nach einem der vorhergehenden Ansprüche, dadurch gekennzeichnet, daß die Imprägnierung des Unterbaus aus gesinterten Metalloxidpartikeln mittels eines Glases erfolgt, das z.B. bis zu 2 % Metalloxide oder Metalle enthält, um der Prothese eine Färbung zu verleihen und so die Farbe des gesinterten Metalloxidgerüstes zu modifizieren.

23. Verfahren nach einem der vorangehenden Ansprüche, dadurch gekennzeichnet, daß die Imprägnierung des Unterbaus aus gesinterten Metalloxidpartikeln mittels eines Glases erfolgt, das hauptsächlich aus einer Mischung von Oxiden besteht, die nach der Verfestigung in einem völlig amorphen Zustand, wobei sie transparent sind, oder in einem mehr oder weniger kristallinen Zustand, wobei sie opaleszent sind, vorliegen.

24. Verfahren nach einem der vorangehenden Ansprüche, dadurch gekennzeichnet, daß die Imprägnierung des gesinterten aus Metalloxiden gebildeten Unterbaus in der Nähe der Oberflächen, die mit dem natürlichen Zahn in Kontakt kommen sollen, dank der Kapillardiffusion des Glases durch das Innere des Unterbaus aus gesinterten Metalloxidpartikeln zustandekommt.

25. Verfahren nach einem der vorangehenden Ansprüche, dadurch gekennzeichnet, daß die Undurchsichtigkeit der erhaltenen Prothese dadurch festgelegt wird, daß für die Imprägnierung des Unterbaus ein Glas benutzt wird, dessen Brechungsindex mehr oder weniger verschieden von dem des Unterbaus ist.

26. Keramikzahnprothese, dadurch gekennzeichnet, daß sie aus einem durchgehend steifen Unterbau mit offenen Poren, der durch Sinterung von Metalloxidpartikeln in fester Phase gebildet ist und dessen Hohlräume mit Glas besetzt sind, besteht.

27. Prothese nach Anspruch 26, dadurch gekennzeichnet, daß sie mit einer Emaille beschichtet ist, die aus einem aluminiumoxidhaltigen alkalischen Borsilikatglas besteht.